# EUROPEAN PATENT SPECIFICATION

(11) **EP 2 316 932 B1**
(45) Date of publication and mention of the grant of the patent: **15.05.2013**
(21) Application number: 09013610.2
(22) Date of filing: 29.10.2009
(51) Int. Cl.: C12N 11/14, C12N 9/18, C12N 9/80

(54) **Enzyme-functionalized supports**
Enzymfunktionalisierte Träger
Supports fonctionnalisés à enzyme

(43) Date of publication of application: 04.05.2011
(73) Proprietor: Gambro Lundia AB, 22010 Lund (SE)
(72) Inventor: Flieg, Ralf, 72414 Rangendingen (DE); Klotz, Manuela, 72379 Hechingen (DE); Storr, Markus, 70794 Filderstadt (DE); Rempfer, Martin, 72810 Gomaringen (DE); Freudemann, Wolfgang, 72379 Hechingen (DE); Winz, Cornelia, 72108 Rottenburg-Weiler (DE); Krause, Bernd, 72414 Rangendingen (DE); Hornung, Markus, 72147 Nehren (DE); Behr, Heinrich, 72379 Hechingen (DE)
(74) Representative: Hornung, Veronika Margot

(56) References cited:
- WO-A1-2005/026224
- TEKE ET AL: "Immobilization of urease using glycidyl methacrylate grafted nylon-6-membranes" PROCESS BIOCHEMISTRY, ELSEVIER, NL, vol. 42, no. 3, 16 February 2007 (2007-02-16), pages 439-443, XP005892730 ISSN: 1359-5113
- RAGHUNATH K ET AL: "PREPARATION AND CHARACTERIZATION OF UREASE IMMOBILIZED ON TO COLLAGEN POLYGLYCIDYL METHACRYLATE GRAFT COPOLYMER" BIOTECHNOLOGY AND BIOENGINEERING, vol. 26, no. 1, 1984, pages 104-109, XP002572231 ISSN: 0006-3592
- PANDURANGA RAO K: "RECENT DEVELOPMENTS OF COLLAGEN-BASED MATERIALS FOR MEDICAL APPLICATIONS AND DRUG DELIVERY SYSTEMS" JOURNAL OF BIOMATERIALS SCIENCE. POLYMER EDITION, VSP, UTRECHT, NL, vol. 7, no. 7, 1 January 1995 (1995-01-01) , pages 623-645, XP002923718 ISSN: 0920-5063
- CHEN BO ET AL: "Candida antarctica lipase B chemically immobilized on epoxy-activated micro- and nanobeads: Catalysts for polyester synthesis" BIOMACROMOLECULES, vol. 9, no. 2, February 2008 (2008-02), pages 463-471, XP002572232 ISSN: 1525-7797

## Description

### Technical Field

The present disclosure relates to enzymes linked to a solid support by a spacer, a method for producing them and the use of such immobilized enzymes.

### Description of the Related Art

Over the last decades, enzymes have found their way into chemical industry, demonstrating their power as catalytic tools. Here, enzymes are used for a plethora of applications; In the paper industry they degrade starch to lower viscosity, aiding sizing and coating paper, they reduce bleach required for decolorizing, smooth fibers and degrade lignin to soften paper. In the last years, the use of enzymes in detergents has been the largest of all enzyme applications. Here, mainly proteases, lipases, amylases and cellulases are employed. They arc able to remove protein or fatty stains as well as residues of starch-based foods. By modifying the structure of cellulose fibers on cotton and cotton blends, the application of cellulases has a color-brightening and softening effect. In the bio-fuel industry, cellulases are used to break down lignocellulose into sugars that can be fermented to yield ethanol which finds application as bio-fuel. In the pharmaceutical industry, enzymes are used for obtaining fine chemicals of high purity as starting materials for the production of pharmaceuticals, e.g., enantiopure amino acids. Enzymes also play a very important role in medical applications, e,g., deoxyribonuclease against cystic fibrosis, asparaginase against acute childhood leukemia, or fibrinolytic streptokinase which is administered to dissolve clots in the arteries of the heart wall after a heart attack. Further, they can be used as analytical tools, e. g. the glucose oxidase for detection and measurement of glucose in blood or urine.

Very important is the role of enzymes in the tood industry. Today, they are used for an increasing range of applications: bakery, cheese making, starch processing and production of fruit juices and other drinks. Here, they can improve texture, appearance and nutritional value, and may generate desirable flavors and aromas. Currently-used food enzymes sometimes originate in animals and plants, but most come from a range of beneficial micro-organisms. In food production, enzymes have a number of advantages: First, they are welcomed as alternatives to traditional chemical-based technology, and can replace synthetic chemicals in many processes. This can allow real advances in the environmental performance of production processes, through lower energy consumption and biodegradability. Second, they are more specific in their action than synthetic chemicals. Processes which use enzymes therefore have fewer side reactions and wasLe by-products, giving higher quality products and reducing the likelihood of pollution. Further, they allow some processes to be carried out which would otherwise be impossible. An example is the production of clear apple juice concentrate, which relies on the use of the enzyme pectinase.

Enzymes are not only the most active known catalysts, but also the most selective. At the same time, they are very sensitive to different conditions, like pH value, temperature or solvent, that can cause the denaturation of the enzymatic structure, thus leading to deactivation of the catalyst. Their use in solution has some disadvantages: First, separation of the enzyme from the reaction mixture and thus recycling is difficult, making the reactions expensive. Second, enzymes are only stable at certain temperatures, pH values, and in certain solvents, which reduces their scope of application.

The immobilization of enzymes removes these disadvantages. It enables the separation of the enzyme from the reaction mixture, and can lower the cost of enzymatic reactions dramatically. This is true for immobilized enzyme preparations which provide a well-balanced overall performance, based on reasonable immobilization yields, low mass transfer limitations and high operational stability. There are many methods available for immobilization which span from binding on pre-fabricated support materials to incorporation into supports prepared in situ. Binding to the support can vary between weak multiple adsorptive interactions and single attachments through strong covalent binding. Which of the methods is most appropriate is usually a matter of the desired applications.

US 4 025 391 A discloses bead-shaped immobilized enzymes produced by adding a solution containing an enzyme and a water-soluble monomer or polymer to a water insoluble fluid to form a mixture having enzyme-containing beads therein, freezing the mixture and polymerizing the frozen mixture by ionizing radiation. The thus obtained beads containing immobilized enzymes can be packed into columns for continuous enzymatic reactions.

EP 0 691 887 B1 discloses activated solid supports for the immobilization of enzymes. As base material, different polymers bearing primary or secondary aliphatic hydroxyl groups are used, on which linear epoxide- or azolactone- containing monomers are then grafted. The immobilization of enzymes occurs by reaction with said functional groups present in the linear polymer chains. Several polymers are disclosed as support material for the immobilization, including polysaccharides based on agarose, cellulose, cellulose derivatives, polymers based on dextran, polymers based on PVA, copolymers of (meth)acrylate derivatives with aliphatic hydroxyl group-containing monomers, diol modified silica gels and copolymers based on polyvinyl.

EP 0 707 064 A2 discloses different methods for immobilizing enzymes on a polymer matrix, especially by physical embedding. First, the enzyme is mixed with a prepolymer, which is water-soluble or emulsifiable in water without auxiliary agents, which exhibits a non-polar backbone to which hydrophilic groups are attached. After evaporation of the solvent, the mixture is heated to 25-70°C to form a polymer matrix in which the enzyme is embedded. Alkyd resins, epoxide resins, isocyanate resins or acrylate resins are suggested as prepolymers.

Teke et al. (2997), Process Biochemistry, Elsevier, NL, Vol. 42 (3), 439-443, describe an immobilized enzyme linked to a solid support and spacer, wherein the solid support is a nylon membrane and the spacer is glycidyl methacrylate. The enzymes which are immobilized are used for the catalytic transformation of certain substrates under heterogeneous conditions. However, the authors do not disclose an immobilized enzyme comprising a spacer linking the enzyme to a solid support, wherein the spacer is linked to the enzyme via a NH function which is part of a carboxyl amide function.

Raghunath et al. (1984), Biotechnology and Bioengineering 26 (1), 104-109, discloses the preparation and characterization of urease which is immobilized onto collagen-poly(glycidyl methacrylate) graft copolymer. However, the authors do not disclose an immobilized enzyme comprising a spacer linking the enzyme to a solid support, wherein the spacer is linked to the enzyme via a NH function which is part of a carboxyl amide function.

Panduranga (1995), Journal of Biomaterials Science, Polymer Edition, VSP, Utrecht, NL, Vol. 7 (7), 623-645, describes recent developments of collagen-based materials and the immobilization of urease and trypsin to collagen graft polymers. However, the authors do not disclose an immobilized enzyme comprising a spacer linking the enzyme to a solid support, wherein the spacer is linked to the enzyme via a NH function which is part of a carboxyl amide function.

Chen Bo et al. (2008), Biomacromolecules 9 (2), 463-471 describes immobilized CALB on epoxy activated poly(methylmethacrylate) *Amberzyme* beads. However, the authors do not disclose solid supports grafted with GMA. They also do not disclose an immobilized enzyme comprising a spacer linking the enzyme to a solid support, wherein the spacer is linked to the enzyme via a NH function which is part of a carboxyl amide function.

WO 2005/026224 A1 discloses a separating material that is obtained by providing a solid support bearing amino-functional groups on its surface, covalently coupling the amino-functional groups to a thermally labile initiator and contacting the solid support with a solution of polymerizable monomers under conditions where thermally initiated graft copolymerization of the monomers takes place, to form a structure of adjacent functional polymer chains on the surface of the support. As solid supports, many polymers are suggested, including polyacrylates, polystyrene, polypropylene and cellulose acetate. The thermally labile radical initiator is coupled by an amide bond to the solid support and is selected among azo compounds or peroxides. The polymerizable monomers are selected from compounds having a polymerizing double bond, preferably (meth)acrylates or acryl amides. The separating material obtained is used for medical applications, e.g., for removing endotoxins from plasma or blood.

The immobilization of enzymes has the advantage of simplifying the recovery of the catalyst, and immobilized enzymes are often more robust towards different reaction conditions than free enzymes. However, immobilized enzymes often show a decreased activity in comparison to enzymes in solution. Thus, there is a continuing need for providing systems for immobilizing enzymes without affecting their activity.

### Summary

It has been found that immobilized enzymes having high catalytic activity can be obtained by linking the enzymes to a solid support via a particular spacer unit. The introduction of the particular spacer unit between the enzyme and the solid support prevents a drop in the enzymatic activity.

The present invention provides enzymes immobilized on a solid support and a method for synthesizing such immobilized enzymes.

Another object of the present invention is the use of such immobilized enzymes in catalytic transformations.

### Brief Description of the Drawings

- Fig. 1: Enzymatic activity of esterase immobilized on dif- ferent support/spacer combinations.
- Fig. 2: Enzymatic activity of urease immobilized on different support/spacer combinations.

### Detailed Description

The present invention provides an immobilized enzyme comprising a) a solid support, b) an enzyme linked to the solid support and c) a spacer, the spacer coupling the enzyme to the solid support and having the general formula: 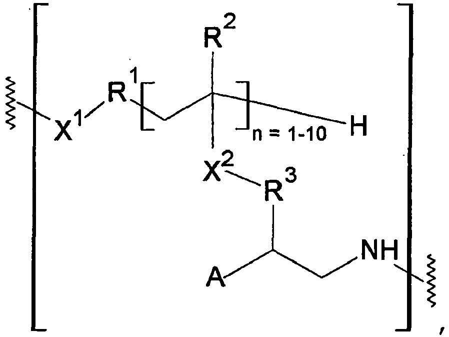 wherein
R¹= aryl, alkyl or heteroalkyl;
R²= hydrogen, methyl;
R³= aryl, heteroalkyl, linear or branched alkyl, or a single bond;
X¹= >C=O, >NH, -N=, =N-, triazole;
X²= -O-, >C=O, >C=S, -C(O)O-, -C(O)NH-, linear or branched alkyl, or a single bond; and
A= hydrogen or OH,
and wherein the spacer is linked to the enzyme by the NH function which is part of a carboxyl amide function.

In a preferred embodiment of the present invention, the solid support is a porous polymeric material. The porosity of the support material provides a large surface area for the contact between the enzyme and the substrate-containing fluid, which is important for a high activity of the enzyme.

The solid support of the present invention may be provided in any suitable form, but preferably is in the form of a membrane, a hollow fiber membrane, a mixed-matrix membrane, a particle bed, a fiber mat, or beads. In one embodiment, the support is in the form of beads. Such beads can, for example, be packed into columns for continuous enzymatic processes.

In another embodiment of invention, the enzyme is immobilized on a mixed-matrix membrane. Mixed-matrix membranes are porous polymeric materials, e.g. in Lhe form of sheets or hollow fibers, in which particles having a variety of functionalities are entrapped, remaining well accessible and maintaining their functionality. Mixed-matrix membranes are described in more detail in US 2006/0099414 A1. A method for the preparation of such mixed-matrix materials comprises providing a mixture of polymeric material and particles in a solvent for the polymeric material and extruding said mixture into a sheet or a hollow fiber and solidifying said sheet or fiber. In addition to their catalytic performance, such membranes can be used to separate desired molecules from the reaction mixture by filtration, for instance macromolecules such as peptides, proteins, nucleic acids or other organic compounds.

The particles can be functionalized before or after they are incorporated into the polymeric material. In one embodiment, the mixed-matrix membrane is functionalized after the steps of extruding and solidifying the membrane. It has been found that the size of the particle, the functionality as well as the amount of particles in the polymer solution have a distinct influence on the ultimate pore structure of the matrix. The smaller the particles, the more spongy the matrix. Furthermore, the accessibility of the particles is significantly improved when the particle content is increased. Thus, such a system enables the formation of a material containing a high concentration of functionalized particles, i.e. immobilized enzymes, the particles being well accessible to a substrate solution.

in another embodiment of invention, the enzyme is immobilized on functional porous multilayer fibers as described in EP 1 627 941 A1. Such fibers comprise a plurality of concentrically arranged porous layers, wherein at least one of the layers comprises functionalized or active particles that are well accessible and maintain their function after preparation. The layer containing high loads of particles can be either the outer or the inner layer. The main function of the other porous layer is to provide mechanical stability to the fiber. IL can further act as a sieve and prevent unwanted compounds or species to come in contact with the functionalized particulate matter. Where it is the inner layer, the second layer can advantageously be a biocompatible material. With the second layer being the outer layer it is possible to reach a particle content of 100 wt% in the inner layer.

Polymers useful in preparing the support materials of the present invention include poly(meth)acrylates like polymethylmcthacrylate (PMMA), polystyrene, polyethylene oxide, cellulose and cellulose derivatives, e.g., cellulose acetate (CA) or regenerated cellulose, polysulfone (PSU), polyethersulfone (PES), polyethylene (PE), polypropylene (PP), polycarbonate (PC), polyacrylonitrile (PAN), polyamide (PA), polytetrafluoroethylene (PTFE), and blends or copolymers of the foregoing, or blends or copolymers with hydrophilizing polymers, preferably with polyvinylpyrrolidone (PVP) or polyethyleneoxide (PEO).

The solid support comprises functional 1 groups on its surface for the covalent coupling to a thermally labile ini-Liator. In one embodiment of this invention, the functional groups are directly coupled to the thermally labile radical initiator. Such groups can be hydroxyl groups, carboxylate groups, ketones, aldehydes, isocyanates, cpoxides, azides which can form esters, amides, imines, urethanes, ureas, amino alcohols or 1,2,3-triazoles, respectively. In another embodiment, the functional groups on the support surface are transformed into groups bearing functional groups suitable for coupling Lo a thermally labile initiator. For example, epoxide groups on the surface of a solid support can be converted to β-hydroxy amines with ammonia. These amino-functional groups can then react with a thermally labile initiator bearing a carboxyl group, forming an amide bond. In another embodiment of this invention, the functional groups are nitriles that can be hydrogenated to yield amino-functional groups or hydrolyzed to yield carboxylates. In a further embodiment, amino-functional groups are provided with a protecting group, e.g., a fluorenylmethoxycarbonyl (fmoc) protecting group. Coupling to the thermally labile initiator is performed upon cleavage of the fmoc-group under basic conditions. The protecting group can be an acid labile group as well, e.g., N-tert-butoxycarbonyl (BOC).

In a particular embodiment, the functional groups on the support surface are amino groups. Preferably, the amino-functional groups on the solid support are primary amino groups, even though secondary amino groups may also be useful. Primary amino groups provide for a higher reactivity. One method to generate amino groups on a solid support is treatment with a reactive plasma, e.g. a plasma generated from a gas mixture comprising ammonia and, optionally, a carrier gas like helium, as described in WO 2006/006918 A1.

The spacer coupling the enzyme to the solid support has the general formula: 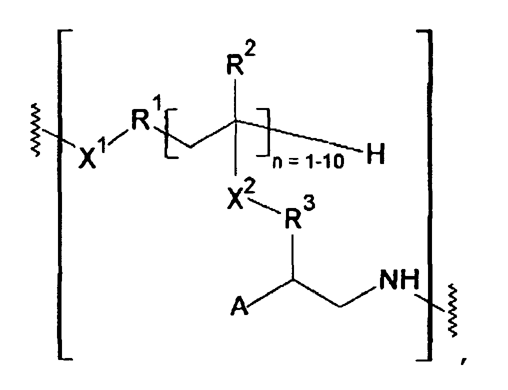 wherein
- R¹=: aryl, alkyl or heteroalkyl;
- R²=: hydrogen, methyl;
- R³=: aryl, heteroalkyl, linear or branched alkyl, or a sin- gle bond;
- X¹=: >C=O, >NH, -N=, =N-, triazole;
- X²-: -O-, >C=O, >C=S, -C(O)O -C(O)NH-, linear or branched alkyl, or a single bond; and
- A=: hydrogen or OH.

The spacer is linked to the solid support by functional group X¹ and is linked to the enzyme by the NH function which usually will be part of a carboxyl amide function formed during the immobilization process by the reaction of a carboxyl group of the enzyme with an amino group of the spacer.

In one embodiment of the invention, the spacer has the formula: 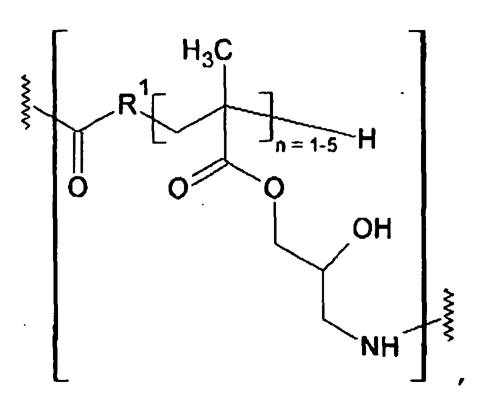 wherein
- R¹=: aryl, alkyl or heteroalkyl.

In one embodiment of the present invention, the spacer is formed on the substrate in a process comprising the steps of
a) providing a solid support having amino-functional groups coupled to the support surface,
b) covalently coupling the amino-functional groups with a thermally labile radical initiator,
c) contacting the support surface with a solution of polymerizable monomers, comprising functional groups which do not take part in radical polymerization, under conditions where thermally initiated graft copolymerization of the monomers takes place, to form polymer chains on the surface of the support,
d) if the polymer chains do not already comprise primary amino groups as functional groups, transforming functional groups of the polymer chains into groups comprising primary amino-functional groups.

In one embodiment of the invention shown in reaction scheme 1, the polymerization initiator is covalently coupled to the support first. Optionally, the amino-group containing supports are reacted with activated esters, e.g., carbodiimide or anhydride activated carboxylic groups of the initiator. The carboxyl groups are preferably activated by the water soluble carbodiimide 1-ethyl-3-(3-dimethylaminopropyl)-carbodiimide (EDAC) which forms active o-acylurea intermediates. After initial activation by EDAC, the carboxyl groups will react with N-hydroxysuccinimide (NHS) to form an active ester, which couples with the primary amino groups on the surface of the support. When using 4,4'-azobis(4-cyanovaleric acid) as initiator, the reaction can be carried out in water or in organic solutions such as DMF, DMSO or toluene.

In a second reaction step, the support comprising the immobilized initiator is contacted with a solution of a radically polymerizable monomer, e.g., glycidyl methacrylate, at an elevated temperature in an inert atmosphere.

In a third reaction step, the epoxide groups are transformed into amino-functional groups by treatment with ammonia.

To produce the immobilized enzyme of the present invention, the amino groups are then coupled to an enzyme, for instance an esterase or an urease, to immobilize the enzyme.

To facilitate reaction with the amino groups, the carboxylic groups of the enzyme can be activated with a carbodiimide, preferably with EDAC, in a buffer solution, e.g., a morpholinoethane sulfonic acid (MES) buffer.

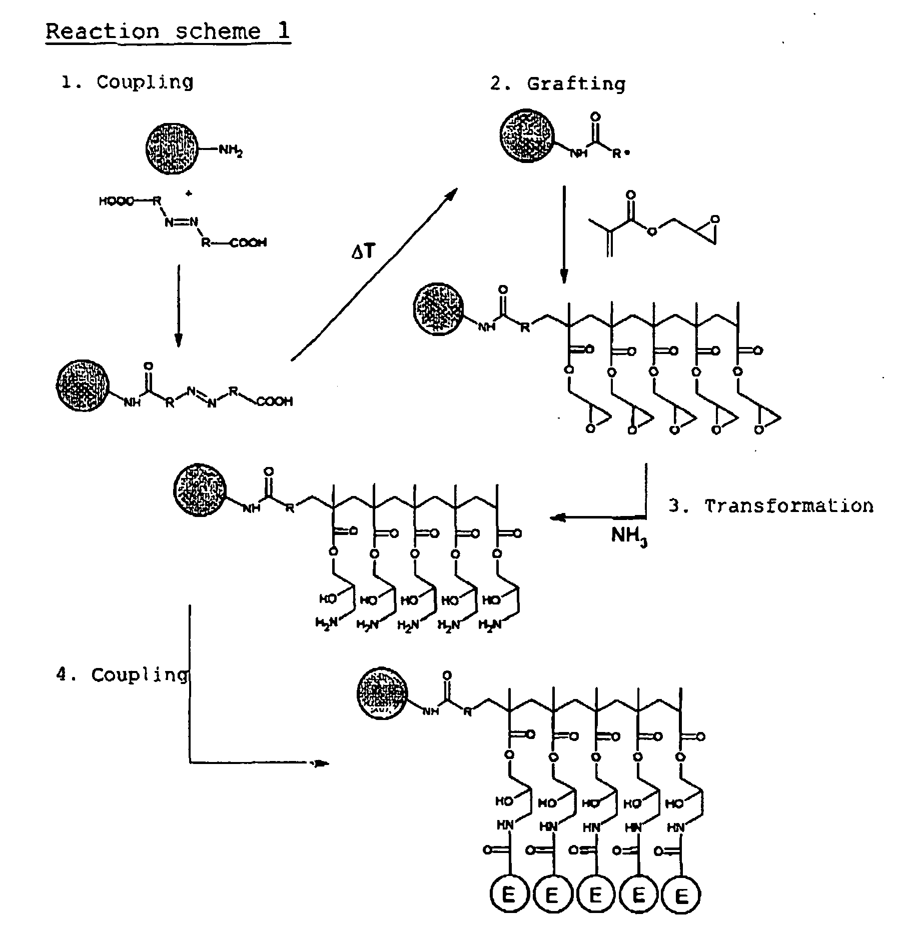

In another embodiment shown in reaction scheme 2, a support having epoxide groups on its surface (e.g., ToyoPearl HW70EC, TOSOH) the epoxide groups on the surface of the solid support are first transformed into β-amino alcohols by treatment with concentrated ammonia solution. The amino-functional groups are then coupled to a thermally labile radical starter as described above.

Subsequently, the support comprising the immobilized initiator is contacted with a solution of a radically polymerizable monomer, e.g., N-(3-aminopropyl)methacrylamide, at an elevated temperature in an inert atmosphere.

The functionalized solid support can be directly coupled to an enzyme, c.g., an urease or an esterase. To facilitate reaction with the amino groups, the carboxylic groups of the enzyme can be activated with a carbodiimide, preferably with EDAC, in a buffer solution, e.g., a morpholinoethane sulfonic acid (MES) buffer.

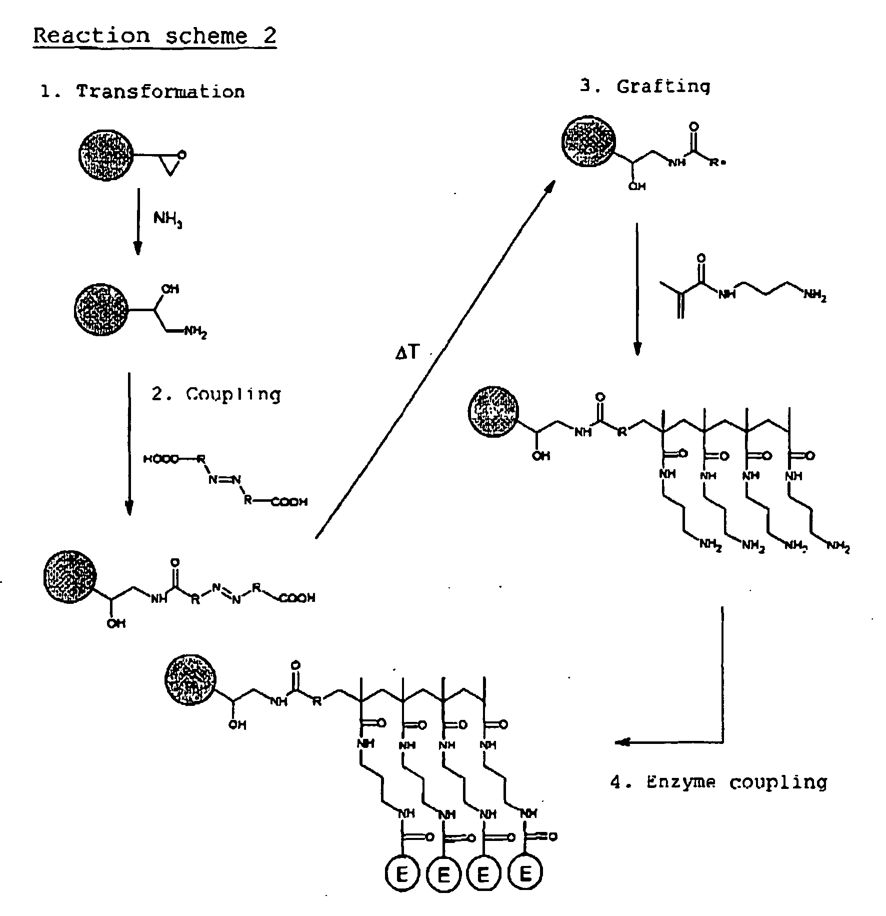

In another embodiment shown in reaction scheme 3, an amino-functionalized solid support (e.g., TentaGel S NH₂, Rapp Polymere) is coupled to a thermally labile radical initiator as described above. In a second reaction step, the support comprising the immobilized initiator is contacted with a solution of a radically polymerizable monomer, e.g., acrylonitrile, at an elevated temperature in an inert atmosphere. The nitrile groups are subsequently reduced to form primary amino functions, e.g., with lithium aluminum hydride (LiAlH₄) or with hydrogen in combination with a metal catalyst. The amino groups arc then coupled to the C-terminus of the enzyme as described above.

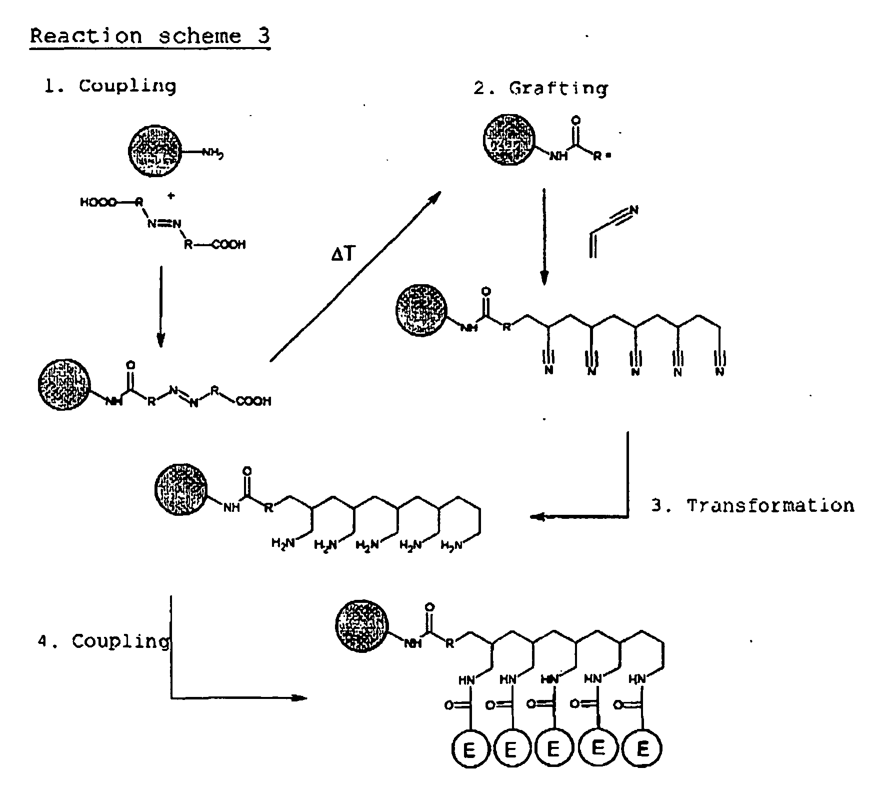

Alternatively, the nitrile can be hydrolyzed to form carboxylate (not shown), thus allowing coupling to the enzyme by the N-terminus using the activating agents mentioned above.

Useful thermally labile radical initiators include compounds which decompose to give free radicals on thermal activation. In one embodiment of the present invention, the thermally labile radical initiator comprises at least one, preferably two carboxylic groups. Preferably, the thermally labile radical initiator is selected from the group comprising azo compounds and peroxides. Most preferred radical initiators are 4,4'-azobis(4-cyanovaleric acid) or 2,2'-azobis[N-(2-carboxyethyl)-2-methylpropionamidine].

In one embodiment of the invention, the carboxylic groups of the radical initiator are activated by a water soluble carbodiimide, e.g., 1-ethyl-3-(3-dimethylaminopropyl)carbodiimide (EDAC) which forms active o-acylurea intermediates. After initial activation, the carboxyl groups will react with e.g. N-hydroxysuccinimide (NHS) to form an active ester, which couples with the primary amino groups on the surface of the support. In another embodiment of the invention, the carboxylic groups of the radical initiator are activated using derivatives of benzotriazole, e. g. IIBTU or HATU.

In another embodiment of the present invention, the thermally labile radical initiator comprises at least one, preferably two amino functions. The amino groups of the radical initiator can react with suitable functional groups on the solid support. In case these groups are epoxides, aldehydes, ketones or isocyanates, no activation agent is needed. If the support bears carboxylic groups, the coupling can be performed by using known procedures, e.g., with EDAC/NHS or HBTU.

In another embodiment of the present invention, the thermally labile radical initiator comprises at least one, preferably two terminal or internal alkynes. The alkyne can then react with an azide function on the support under mild conditions to yield a 1,2,3-triazole. As catalyst, Cu(I)-salts or Cu(II)-salts in combination with a reducing agent, e.g., tris(benzyltriazolylmethyl)amine (TBTA), can be used. The reaction can be carried out in a variety of solvents, for instance, mixtures of water and (partially) water-miscible organic solvents including DMSO, DMF, acetone, and alcohols like t-BuOH.

An advantage of the process of the present invention lies in the covalent coupling of the radical initiator to the functional groups on the solid support. Thereby, the occurrence of homopolymerization in the reaction solution is avoided or at least minimized. The radical initiator, which is bound to the solid support, forms radicals upon temperature increase, and parL of the radical initiator structure becomes part of the polymer chains, which are formed from the solid support surface. The polymer chains of the present invention develop from the surface of the substrate without the formation of undesired cross-linkages between the chains, thus the process of the present invention is considered to provide a very "clean" chemistry.

Another advantage of the present, invention is based on the use of thermally labile radical initiators. These can be chosen to ensure mild reaction conditions and avoid additional reactants which may react with the support or the monomers in an undesired manner.

The temperatures to initiate radical formation of useful radical initiators typically lie within the range of 50°C to 120°C, preferably in the range of 70°C Lo 100°C. A useful temperature range of the polymerization reaction is from the 10 hour half-life temperature of the radical initiator to about 20 Lo 25 degrees above that 10 hour half-life temperature. By adjusting the reaction temperature, it is further possible to very precisely control the polymerization reaction, e.g., onset of the reaction, reaction rate, degree of polymerization, etc.

The monomers useful to form the polymer chains of the spacer linking the enzyme to the support surface are compounds havinq a polymerizable double bond. Since the coupling to the enzyme occurs either through its amino function or its carboxylic group, the monomer additionally comprises a suitable functional group for coupling to one of the enzymatic termini. Suitable functional groups are primary or secondary amines or hydroxyl functions for reaction with the C-terminus and carboxyl groups, ketones, aldehydes, epoxide groups or isocyanates for reaction with the N-terminus. Alternatively, the monomer can comprise functional groups that are able to couple to the enzyme after transformation into active functional groups. Such functional groups are nitriles, nitro compounds, alcohols, ethers, hemi-acetals or acetals. The monomer can also comprise amino functions carrying protecting groups like N-tert-butoxycarbonyl (BOC) or 2-fluorenylmethoxycarbonyl (Fmoc) which are cleaved before coupling the spacer to the enzyme.

In one embodiment, the monomers are selected from glycidyl acrylate, glycidyl methacrylate (GMA), vinyl glycidyl ether, and vinyl glycidyl urethane. In a particular embodiment, the monomer is glycidyl methacrylate. Before the enzyme is coupled to the spacer, the epoxide groups are preferably transformed into amino-functional groups, e.g., using an aqueous solution of ammonia.

The polymerization reaction can be performed using a single one of the above-mentioned monomers, or it can be carried out using two or more different monomers.

In one embodiment of invention, the polymer chain grafted on the solid support comprises, on average, 1 to 10 monomer units, for instance 1 to 5 monomer units, or even 2 to 5 monomer units.

The spacer of the present invention comprises a part deriving from the thermally labile initiator, covalently coupled to the solid supports, and a part deriving from the polymerizable monomer, comprising functional groups that are inert under polymerizing conditions, but are reactive towards the functional groups of the enzyme termini and become covalently coupled to Lhe enzyme.

The enzyme coupled to the spacer can be chosen among the known classes of enzymes. The enzyme can be an oxidoreductase, a transferase, a hydrolase, a lyase, an isomerase or a ligase. Preferred enzymes are a urease or an esterase. In one embodiment, only one type of enzyme is immobilized on the support. In another embodiment, a mixture of two or more enzymes is immobilized. Such systems can be of interest if a product of a transformation by a first enzyme becomes a substrate for a second enzyme.

Coupling to the spacer can occur either by the C-terminus or by the N-terminus of the enzyme. In one embodiment, the C-terminus is coupled to the support. In a particular embodiment, the C-terminus is activated by a water-soluble carbodiimide, e.g., 1-ethyl-3-(3-dimethylaminopropyl)carbodiimide (EDAC), which forms active o-acylurea intermediates. After initial activation, the carboxyl groups will react with, e.g., N-hydroxysuccinimide (NHS) to form an active ester, which couples with the primary amino groups of the spacer.

In another embodiment of the invention, the C-terminus is activated using derivatives of benzotriazole, e. g. HRTU or HATU. An advantage of these coupling reagents is the direct formation of an active ester by reaction with a carboxyl function, so that no second activation reagent like N-hydroxysuccinimide (NHS) is needed. Because of their pH sensitivity, coupling reactions with enzymes are normally carried out in a buffer system, e.g., morpholinoethane sulfonic acid (MES) or a phosphate buffer.

It will be understood that the features mentioned hereinafter can be used not only in the combination specified but also in other combinations or on their own, without departing from the scope of the present invention.

The present invention will now be described in more detail in the examples below. It is to be understood that the examples are not intended to limit the scope of the present invention.

### Examples

### Example 1 Coupling of 4,4'-azobis(4-cyanovaleric acid) onto macroporous acrylic beads.

500 g oxirane acrylic resin beads (Toyo Pearl HW70EC, Tosoh Corp.) having an average epoxy group content of 4.0 mmol/q were aminated with 2.5 L conc. ammonia solution (32 wt%) over 16 hours aL room temperature. After washing with distilled water, 500 g beads were resuspended in 4 L of 0.1 M sodium hydroxide solution and 44 g 4,4'-azobis(4-cyanovaleric acid), 64 g EDAC and 64 g NHS were added. The batch was agitated over 16 hours at room temperature and afterwards rinsed with water.

### Example 2 Coupling of 4,4'-azobis(4-cyanovaleric acid) onto microporous hollow fiber membranes

A bundle of polyethersulfone/polyvinylpyrrolidone hollow fiber membranes (105 fibers, 25 cm long, inner diameter 320 µm, outer diameter 420 µm, mean pore diameter 0.3 µm, functionalized with approximately 15 µmol/g primary amino groups by plasma treatment as described in WO 2006/006918 A1) was incubated with 13.8 g 4,4'-azobis(4-cyanovaleric acid) and 20.0 g NHS in 1125 mL 0.1 M NaOH. Then 20.0 g EDAC dissolved in 125 ml 0.1 M NaOH was added and the mixture was agitated for 16 h at room temperature. The excess reagents were subsequently removed by washing repeatedly with water.

### Example 3 Graft polymerization of beads with glycidyl methacrylate

a) 500 g of the beads obtained in Example 1 were reacted in a solution of 96 g qlycidyl methacrylatc in 4 L DMF in a three-necked flask. The reaction was performed with gentle stirring at 75°C for 16 hours in an atmosphere of nitrogen (reflux condenser), The derivatized beads were then thoroughly rinsed with water and dried overnight at 40°C in a vacuum drying oven.
b) 500 g of the beads obtained in a) were treated with 2.5 L conc. ammonia solution (32 wt%) over 16 hours at room temperature to convert the epoxy groups into primary amino groups. The beads were subsequently washed with water and dried.

### Example 4 Graft polymerization of microporous membranes with glycidyl methacrylate

a) The bundle of membranes obtained in Example 2 was reacted in a reaction solution of 23.6 g glycidyl methacrylate in 1250 mL isopropanol in a three-necked flask. The reaction was performed with gentle stirring at 75°C for 16 hours in an atmosphere of nitrogen.
b) The derivatized fiber bundles (each comprising 105 membranes) were potted into module housings. For the conversion of the epoxy groups into primary amino groups the modules were filled with 2 M ammonia solution. After a reaction time of 4 hours at room temperature the membranes were subsequently washed with water and dried.

### Example 5 Esterase immobilized on functionalized beads

Esterase: E.C. number: 3.1.1.42, CES (CD: 60756), Kikkoman Corporation, 376-2 Kamihanawa Noda-City, Chiba, Japan.

The enzyme coupling was performed in 0.1 M MES buffer at pH 5.4 (MES = morpholino-ethane sulfonic acid). The esterase was dissolved in a concentration of 90 mg/mL and EDAC as a catalyst. for the amid-binding was dissolved in a concentration of 5 mg/mL. Per 1 g of dry functionalized beads, 5 mT, of the coupling solution was added. The coupling was performed for 24 hours at room temperature. The reaction vials were gently shaken during this time. The enzyme beads were then washed with MES buffer, water, and 67 mM phosphate buffer (pH 7.4). The esterase beads were stored in the buffer solution at 5°C.

The following four different types of esterase beads were tested for enzymatic activity:
[1] TOSOH HW70EC (epoxy groups) + Esterase
[2] Beads obtained in Example 3a) + Esterase
[3] TOSOH HW70EC + ammonia (-> amino groups) + Esterase
[4] Reads obtained in Example 3b) + Esterase

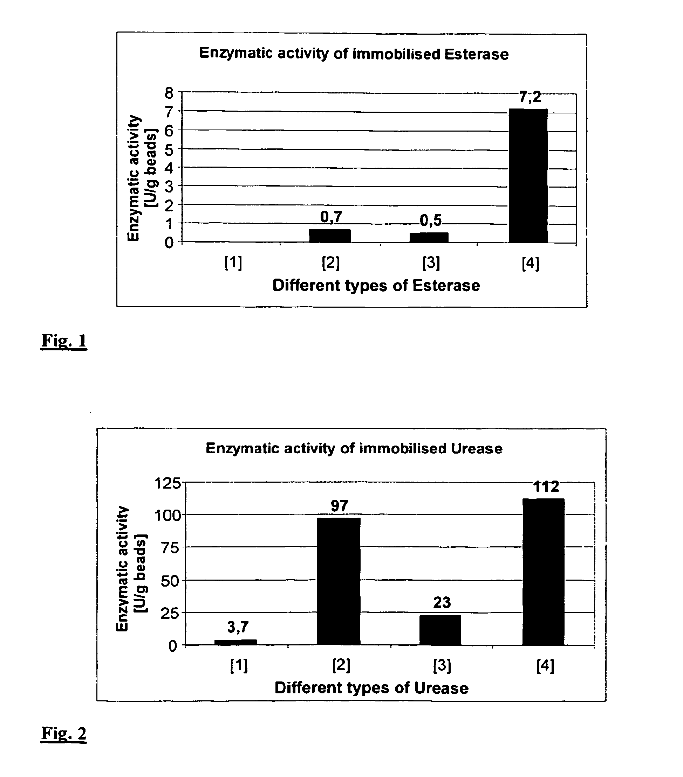

For the detection of the enzymatic activity, about 10 mg of esterase beads were incubated with a defined amount of the substrate (0.5 mL of an aqueous 0.5 M solution of chlorogenic acid). After 30 minutes at 30°C, the enzymatic conversion was stopped by adding 10 mL of ethanol (80%). The decrease in substrate concentration was measured via photometry at OD350. The results are summarized in Figure 1. The enzymatic activities are given in U/g beads.

### Example 6 Urease immobilized on functionalized beads

Urease: E.C. number: 3.5.1.5, Cat: URE2, Biozyme Laboratories Ltd., Unit 6, Gilchrist-Thomas Ind. Est., Blaenavon, Gwent NP4 9RL, UK

The same set-up of different functionalized beads as in Example 5 was used for screening. The following systems were tested for enzymatic activity:
[1] TOSOH HW70EC (epoxy groups) + Urease
[2] Beads obtained in Example 3a) + Urease
[3] TOSOH HW70EC + ammonia (-> amino groups) + Urease
[4] Beads obtained in Example 3b) + Urease

The enzyme coupling was performed in 0.1 M phosphate buffer (plus 1 M EDTA) at pH 5.3. The urease was dissolved in a concentration of 1 mg/mL. Per 1 g of dry functionalized beads, 40 mL of the coupling solution was added. The coupling was performed for 24 hours at room temperature. The reaction vials were gently shaken during this time. The enzyme beads were subsequently washed with water and 0.5 M sodium chloride solution. Finally, the beads were siphoned off and stored at 5°C.

For the detection of the enzymatic activity, about 50 mg of urease beads were incubated with a defined amount of the substrate (45 mL of an aqueous solution of urea with a concentration of 3 g/L). After 60 minutes aL 37°C, a sample was taken from the supernatant. The formation of ammonia was measured via photometry. The results are summarized in Figure 2. The enzymatic activities are given in U/g beads.

### Example 7 Urease immobilized on functionalized membranes

Urease: E.C. number: 3.5.1.5, Cat: URE2, Biozyme Laboratories Ltd., Unit 6, Gilchrist-Thomas Ind. Est., Blaenavon, Gwent NP4 9RL, UK

Urease was immobilized on membranes obtained as described in Example 4. The enzyme coupling was performed in 0.1 M PBS buffer at pH 5.3 (plus 1 M EDTA). The urease was dissolved in a concentration of 5 mg/mL. Per membrane module (small housing with 80 potted fibers) 40 mL of coupling solution were circulated for 24 hours at room temperature. Afterwards the enzyme membranes were washed with 0.5 M sodium chloride solution and with water. The urease membranes were stored at 5 °C.

For the detection of the enzymatic activity, an aqueous urea solution was circulated through the urease membrane module. The substrate was provided at a concentration of 1 g/L and the pool had a volume of 200 mL. The activity test was performed at 37°C. The formation of ammonia was measured via photometry. The samples showed depletion of substrate over the four hours treatment time. The immobilized urease showed an enzymatic activity of 527 U/g beads after 30 min of perfusion, and 421 U/g beads after 1 hour.

### Example 8 Esterase immobilized on functionalized membranes

Esterase: E.C. number: 3.1.1.42, CES (CD: 60756), Kikkoman Corporation, 376-2 Kamihanawa Noda-City, Chiba, Japan.

Esterase was immobilized on membranes obtained as described in Example 4. The enzyme coupling was performed in 0.1 M MES buffer at pH 5.4 (MES = morpholino-cthane sulfonic acid). The esterase was dissolved in a concentration of 90 mg/mL, and EDC as a catalyst for the amide-binding was dissolved in a concentration of 5 mg/mL. Per membrane module (small housing with 10b potted fibers), 25 mL of coupling solution were circulated for 24 hours at room temperature. The membranes were subsequently washed with 0.1 M MES buffer and with water. The esterase membranes were stored at 5 °C.

For the detection of the enzymatic activity, an aqueous solution of chlorogenic acid was pumped single-pass through the esterase membrane module at a flow rate of 1.2 mL/min, The substrate was provided at a concentration of 0.07 g/L. The activity test was performed at 30°C. The hydrolysis of chlorogenic acid was measured via photometry (OD350). After 1 hour of perfusion, a stable enzymatic activity of 0.3 U/g fibers was measured.

### Example 9 Esterase immobilized on functionalized membranes

Esterase: E.C. number: 3.1.1.42, CES (CD: 60756), Kikkoman Corporation, 376-2 Kamihanawa Noda-City, Chiba, Japan.

Esterase was immobilized on membranes obtained as described in Example 4. The enzyme coupling was performed in 0.1 M MES buffer at pH 5.4 (MES = morpholino-ethane sulfonic acid). The esterase was dissolved in a concentration of 90 mg/mL, and EDC as a catalyst for the amide-binding was dissolved in a concentration of 5 mg/mL. Per membrane module (housing with approx. 1200 potted fibers), 200 mL of coupling solution were circulated for 24 hours at room temperature. The membranes were subsequently washed with 0.1 M MES buffer and with water. The esterase membranes were stored aL 5 °C.

For the detection of the enzymatic activity, an aqueous solution of chlorogenic acid was circulated through the esterase membrane module. The substrate was provided at a concentration of 1.8 g/L and the pool had a volume of 200 mL. The activity test was performed at 30°C. The hydrolysis of chlorogenic acid was measured via photometry (OD350). After 30 min of perfusion, the immobilized esterase showed an enzymatic activity of 2.8 U/g fibers. After 1 hour of perfusion, the immobilized esterase showed an enzymatic activity of 1.8 U/g fibers.

### Example 10 Esterase immobilized on mixed-matrix membranes

Esterase: E.C. number: 3.1.1.42, CES (CD: 60756), Kikkoman Corporation, 376-2 Kamihanawa Noda-City, Chiba, Japan.

Beads from Example 3 were added Lo a polymer solution comprising PES and PVP and mixed-matrix hollow fiber membranes were spun from the polymer solution. The enzyme coupling was performed in 0.1 M MES buffer at pH 5.4 (MES = morpholino-ethane sulfonic acid). The esterase was dissolved in a concentration of 90 mg/mL and EDC as a catalyst for the amide-binding was dissolved in a concentration of 5 mg/mL. Per membrane module (small housing with 82 potted fibers), 25 mL of coupling solution were circulated for 24 hours at room temperature. The membranes were subsequently washed with 0.1 M MES buffer and with water. The esterase membranes were stored at 5 °C.

For the detection of the enzymatic activity, an aqueous solution of chlorogenic acid was pumped single-pass through the esterase membrane module at a flow rate of 1.2 mL/min. The substrate was provided at a concentration of 0.7 g/L. The activity test was performed at 30°C. The hydrolysis of chlorogenic acid was measured via photometry (OD350). After 3 hours of perfusion, a stable enzymatic activity of 0.8 U/q fibers was measured.

## Claims

1. An immobilized enzyme comprising
a) a solid support,
b) an enzyme linked to the solid support,
c) a spacer, the spacer coupling the enzyme to the solid support and having the general formula: 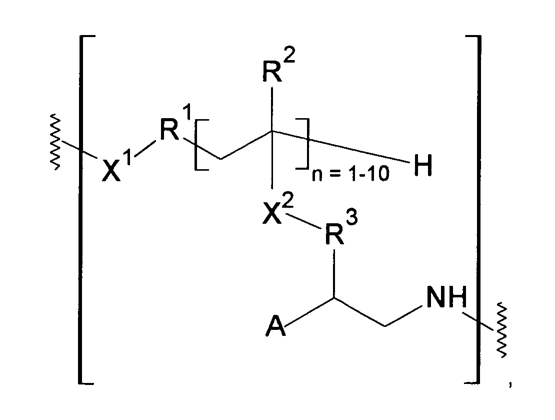
wherein
R¹= aryl, alkyl or heteroalkyl;
R²= hydrogen, methyl;
R³= aryl, heteroalkyl, linear or branched alkyl, single bond;
X¹= >C=O, >NH, -N=, =N-, triazole;
X²= -O-, >C=O, >C=S, -C(O)O-, -C(O)NH-, linear or branched alkyl, single bond; and
A= hydrogen or OH,
and wherein the spacer is linked to the enzyme by the NH part of a carboxyl amide functional group.

2. The immobilized enzyme of claim 1, wherein the solid support is a porous polymeric material.

3. The immobilized enzyme of claims 1 to 2, wherein the solid support is in the form of a membrane, a hollow fibre membrane, a mixed-matrix membrane, a particle bed, a fibre mat, or beads.

4. The immobilized enzyme of claims 1 to 3, wherein the enzyme is an esterase.

5. The immobilized enzyme of claims 1 and 3, wherein the enzyme is a urease.

6. The immobilized enzyme of claims 1 to 5, wherein the spacer has the formula: 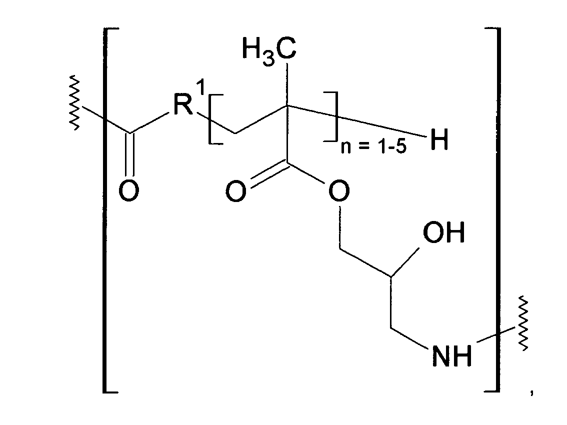 wherein
R¹= aryl, alkyl or heteroalkyl.

7. A method for the production of immobilized enzymes comprising the steps of
a) providing a solid support having amino-functional groups coupled to the support surface,
b) covalently coupling the amino-functional groups with a thermally labile radical initiator,
c) contacting the support surface with a solution of polymerizable monomers, comprising functional groups which do not take part in radical polymerization, under conditions where thermally initiated graft copolymerization of the monomers takes place, to form polymer chains on the surface of the support,
d) if the polymer chains do not already comprise primary amino-functional groups, transforming the functional groups in the polymer chains into groups comprising primary amino-functional groups,
e) coupling the enzyme to amino-functional groups of the polymer chains.

8. The method of claim 7, wherein the solid support is a porous polymeric material.

9. The method of any of claims 7 and 8, wherein the solid support is in the form of a membrane, a hollow fibre membrane, a mixed-matrix membrane, a particle bed, a fibre mat, or beads.

10. The method of any claim 7 to 9, wherein the functional groups of the polymerizable monomer are epoxide groups.

11. The method of any claim 7 to 10, wherein the thermally labile radical initiator comprises at least one carboxylic group.

12. The method of any claim 7 to 11, wherein the thermally labile radical initiator is selected from azo compounds or peroxides.

13. The method of any claim 7 to 12, wherein the thermally labile radical initiator is 4,4'-azobis-(4-cyanovaleric acid) or 2,2'-azobis-[N-(2-carboxyethyl)-2-methylpropionamide].

14. The method of any claim 7 to 13, wherein the polymerizable monomer is glycidyl methacrylate (GMA).

15. In vitro use of immobilized enzymes of any of claims 1 to 6, or produced by the method of any one of claims 7 to 14, for catalytic transformations of substrates under heterogeneous conditions.

## Patentansprüche

1. Immobilisiertes Enzym, welches das Folgende umfasst:
a) einen festen Träger,
b) ein Enzym, welches an den festen Träger gebunden ist,
c) einen Spacer, wobei der Spacer das Enzym mit dem festen Träger verbindet und die folgende allgemeine Formel aufweist: 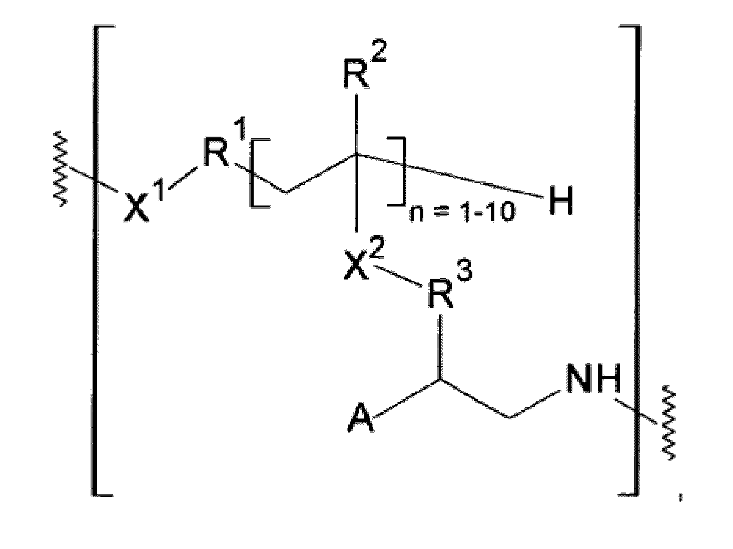
wobei
R¹ = Aryl, Alkyl oder Heteroalkyl;
R² = Wasserstoff, Methyl;
R³ = Aryl, Heteroalkyl, lineares oder verzweigtes Alkyl, Einfachbindung; X¹ = >C=O, >NH, -N=, =N-, Triazol;
X² = -O-, >C=O, >C=S, -C(O)O-, -C(O)NH-, lineares oder verzweigtes Alkyl, Einfachbindung und
A = Wasserstoff oder OH ist
und wobei der Spacer durch die NH-Gruppe, die Teil einer funktionellen Carboxylamidgruppe ist, mit dem Enzym verbunden ist.

2. Immobilisiertes Enzym nach Anspruch 1, wobei es sich bei dem festen Träger um ein poröses Polymermaterial handelt.

3. Immobilisiertes Enzym nach Anspruch 1 bis 2, wobei der feste Träger in Form einer Membran, einer Hohlfasermembran, einer Mischmatrixmembran, eines Partikelbetts, einer Fasermatte oder in Form von Kügelchen vorliegt.

4. Immobilisiertes Enzym nach Anspruch 1 bis 3, wobei es sich bei dem Enzym um eine Esterase handelt.

5. Immobilisiertes Enzym nach Anspruch 1 und 3, wobei es sich bei dem Enzym um eine Urease handelt.

6. Immobilisiertes Enzym nach Anspruch 1 bis 5, wobei der Spacer die folgende Formel aufweist: 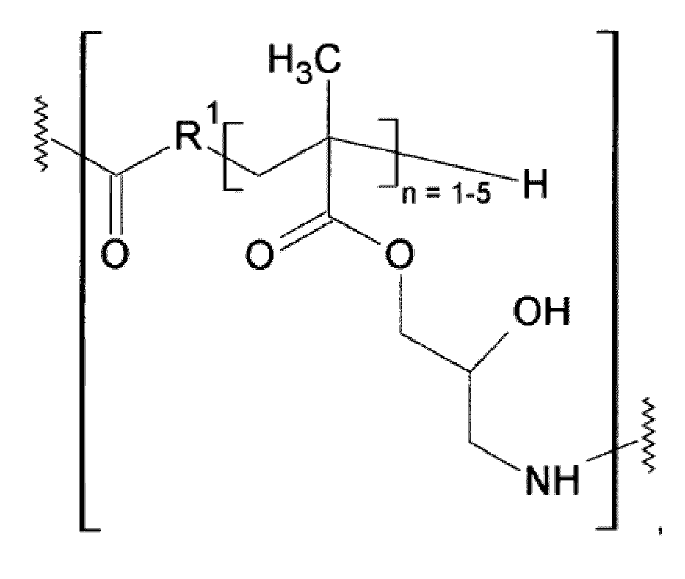 wobei
R¹ = Aryl, Alkyl oder Heteroalkyl.

7. Verfahren zur Herstellung immobilisierter Enzyme, welches die folgenden Schritte umfasst:
a) Bereitstellen eines festen Trägers, welcher Aminogruppen aufweist, die mit der Trägerfläche verbunden sind,
b) kovalentes Verbinden der Aminogruppen mit einem thermisch labilen Radikalstarter,
c) In-Kontakt-Bringen der Trägerfläche mit einer Lösung polymerisierbarer Monomere, welche funktionelle Gruppen umfassen, die nicht an einer radikalischen Polymerisation teilnehmen, unter Bedingungen, bei denen eine thermisch initiierte Pfropfcopolymerisation der Monomere stattfindet, um Polymerketten auf der Fläche des Trägers zu bilden,
d) wenn die Polymerketten nicht bereits primäre Aminogruppen umfassen, Umwandeln der funktionellen Gruppen in den Polymerketten in Gruppen, welche primäre Aminogruppen umfassen,
e) Verbinden des Enzyms mit Aminogruppen der Polymerketten.

8. Verfahren nach Anspruch 7, wobei es sich bei dem festen Träger um ein poröses Polymermaterial handelt.

9. Verfahren nach einem der Ansprüche 7 und 8, wobei der feste Träger in Form einer Membran, einer Hohlfasermembran, einer Mischmatrixmembran, eines Partikelbetts, einer Fasermatte oder in Form von Kügelchen vorliegt.

10. Verfahren nach einem der Ansprüche 7 bis 9, wobei es sich bei den funktionellen Gruppen des polymerisierbaren Monomers um Epoxidgruppen handelt.

11. Verfahren nach einem der Ansprüche 7 bis 10, wobei der thermisch labile Radikalstarter mindestens eine Carboxylgruppe umfasst.

12. Verfahren nach einem der Ansprüche 7 bis 11, wobei der thermisch labile Radikalstarter aus Azoverbindungen und Peroxiden ausgewählt ist.

13. Verfahren nach einem der Ansprüche 7 bis 12, wobei es sich bei dem thermisch labilen Radikalstarter um 4,4'-Azobis-(4-cyanovaleriansäure) oder 2,2'-Azobis-[N-(2-carboxyethyl)-2-methylpropionamid] handelt.

14. Verfahren nach einem der Ansprüche 7 bis 13, wobei es sich bei dem polymerisierbaren Monomer um Glycidylmethacrylat (GMA) handelt.

15. In-vitro-Verwendung immobilisierter Enzyme nach einem der Ansprüche 1 bis 6 oder In-vitro-Verwendung immobilisierter Enzyme, welche über das Verfahren nach einem der Ansprüche 7 bis 14 hergestellt werden, für katalytische Umwandlungen von Substraten unter heterogenen Bedingungen.

## Revendications

1. Enzyme immobilisée comprenant :
a) un support solide,
b) une enzyme liée au support solide,
c) un espaceur, l'espaceur couplant l'enzyme au support solide et ayant la formule générale suivante : 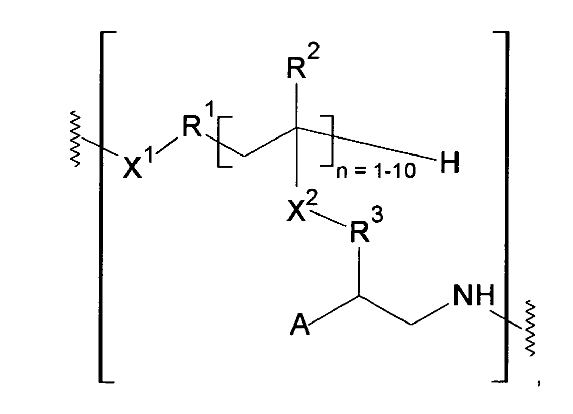
dans laquelle
R¹ = groupe aryle, alkyle ou hétéroalkyle ;
R² = atome d'hydrogène, groupe méthyle ;
R³ = groupe aryle, hétéroalkyle, alkyle linéaire ou ramifié, liaison simple ;
X¹ = >C=O, >NH, -N=, =N-, triazole ;
X² = -O-, >C=O, >C=S, -C(O)O-, -C(O)NH-, groupe alkyle linéaire ou ramifié, liaison simple ;
et
A = atome d'hydrogène ou groupe OH
et dans laquelle l'espaceur est lié à l'enzyme par la partie NH d'un groupe fonctionnel carboxylamide.

2. Enzyme immobilisée selon la revendication 1, le support solide étant un matériau polymère poreux.

3. Enzyme immobilisée selon les revendications 1 à 2, le support solide étant sous la forme d'une membrane, d'une membrane à fibres creuses, d'une membrane à matrice mixte, d'un lit de particules, d'un mat de fibres ou de perles.

4. Enzyme immobilisée selon les revendications 1 à 3, l'enzyme étant une estérase.

5. Enzyme immobilisée selon les revendications 1 et 3, l'enzyme étant une uréase.

6. Enzyme immobilisée selon les revendications 1 à 5, l'espaceur ayant pour formule : 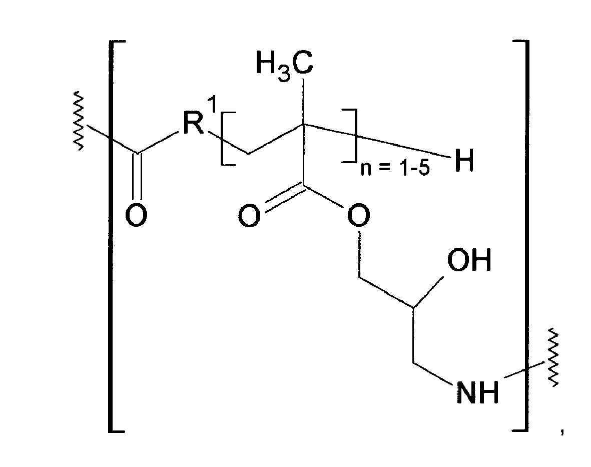 dans laquelle
R¹ = groupe aryle, alkyle ou hétéroalkyle.

7. Procédé de production d'enzymes immobilisées comprenant les étapes consistant à
a) apporter un support solide ayant des groupes amino-fonctionnels couplés à la surface du support,
b) coupler de manière covalente les groupes amino-fonctionnels avec un initiateur radicalaire thermolabile,
c) mettre en contact la surface du support avec une solution de monomères polymérisables, comprenant des groupes fonctionnels qui ne participent pas à la polymérisation radicalaire, dans des conditions dans lesquelles se déroule une copolymérisation des monomères avec greffage initiée thermiquement, afin de former des chaînes polymères à la surface du support,
d) si les chaînes polymères ne comprennent pas déjà des groupes amino-fonctionnels primaires, transformer les groupes fonctionnels des chaînes polymères en des groupes comprenant des groupes amino-fonctionnels primaires,
e) coupler l'enzyme aux groupes amino-fonctionnels des chaînes polymères.

8. Procédé selon la revendication 7, dans lequel le support solide est un matériau polymère poreux.

9. Procédé selon l'une quelconque des revendications 7 et 8, dans lequel le support solide est sous la forme d'une membrane, d'une membrane à fibres creuses, d'une membrane à matrice mixte, d'un lit de particules, d'un mat de fibres ou de perles.

10. Procédé selon l'une quelconque des revendications 7 à 9, dans lequel les groupes fonctionnels du monomère polymérisable sont des groupes époxyde.

11. Procédé selon l'une quelconque des revendications 7 à 10, dans lequel l'initiateur radicalaire thermolabile comprend au moins un groupe carboxylique.

12. Procédé selon l'une quelconque des revendications 7 à 11, dans lequel l'initiateur radicalaire labile thermiquement est choisi parmi des composés azoïques ou des peroxydes.

13. Procédé selon l'une quelconque des revendications 7 à 12, dans lequel l'initiateur radicalaire labile thermiquement est le 4,4'-azo-bis-(acide 4-cyanovalérique) ou le 2,2'-azo-bis-[N-(2-carboxyéthyl)-2-méthylpropionamide].

14. Procédé selon l'une quelconque des revendications 7 à 13, dans lequel le monomère polymérisable est le méthacrylate de glycidyle (GMA).

15. Utilisation *in vitro* d'enzymes immobilisées selon l'une quelconque des revendications 1 à 6, ou produites via le procédé selon l'une quelconque des revendications 7 à 14, pour des transformations catalytiques de substrats dans des conditions hétérogènes.
